(19) Europäisches Patentamt
European Patent Office
Office européen des brevets

(11) Publication number: **0 095 320**
**A1**

(12) # EUROPEAN PATENT APPLICATION

(21) Application number: 83302844.2

(22) Date of filing: 19.05.83

(51) Int. Cl.³: C 12 P 37/00
C 12 P 35/08, C 12 P 17/10
C 07 D 499/00
//C12P35/00, C12R1/465

(30) Priority: 21.05.82 GB 8214819
21.05.82 GB 8214818
29.07.82 GB 8221952
29.07.82 GB 8221953

(43) Date of publication of application:
30.11.83 Bulletin 83/48

(84) Designated Contracting States:
CH DE FR GB IT LI NL

(71) Applicant: BEECHAM GROUP PLC
Beecham House Great West Road
Brentford Middlesex(GB)

(72) Inventor: Hood, John Dick
Benmore Rowly Drive
Cranleigh Surrey(GB)

(72) Inventor: Elson, Anna Louisa
8 Wimblehurst Road
Horsham Sussex(GB)

(74) Representative: Hesketh, Alan, Dr. et al,
European Patent Attorney Beecham Pharmaceuticals
Great Burgh Yew Tree Bottom Road
Epsom, Surrey, KT18 5XQ(GB)

(54) **A process for the preparation of beta-lactam compounds.**

(57) A process for the preparation of a β-lactam having the partial structure (I):

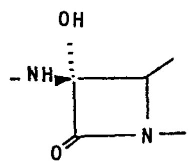

(I)

which process comprises reacting a β-lactam having the partial structure (II):

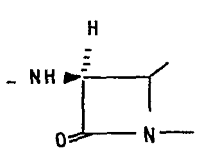

(II)

with an oxygenase enzyme. The β-lactam having partial structure (II) may be isolated or converted to the corresponding β-lactam having a methoxy group by treatment with a methylating agent such as, for example, a methyl transferase enzyme.

EP 0 095 320 A1

# THE PROCESS FOR THE PREPARATION OF β-LACTAM COMPOUNDS

This invention relates to β-lactam compounds and in particular to a process for preparing hydroxy substituted β-lactam derivatives. The invention also relates to novel β-lactam derivatives and to their use as chemical intermediates.

J.O'Sullivan et al in Biochem J., 1979, 179, 47-52 and Biochem J. 1980, 186, 613-616 report the conversion using cell-free extracts of Streptomyces clavuligerus of the compound of formula (A):

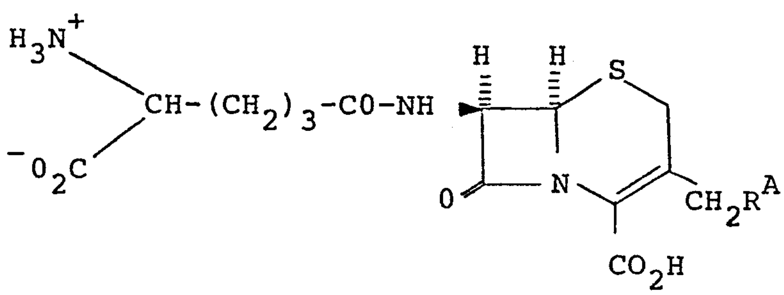

(A)

wherein $R^A$ represents $OCONH_2$, $OCOCH_3$ or H into the compound of formula (B):

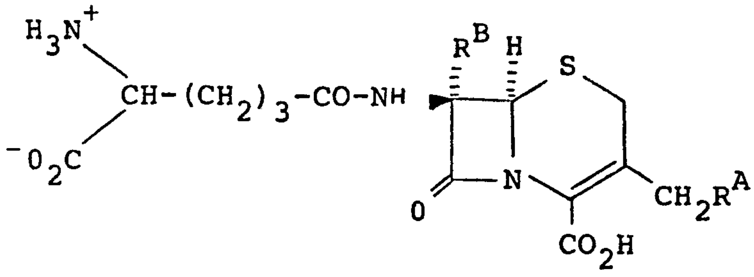

(B)

wherein $R^A$ is as described above and $R^B$ represents a methoxy group. O'Sullivan suggested that the 7α-methoxy group originates from an oxygenation at C-7 and methylation of a hydroxylated intermediate, ie the compound of formula B wherein $R^B$ is hydroxy, by a methyltransferase.

O'Sullivan et al. further report that using their enzyme system no evidence was obtained for the incorporation of a methoxyl group into deacetylcephalosporin C, cefuroxime, cephalothin, penicillin N, clavulanic acid or nocardicin A.

We have now found a process for the preparation of β-lactams having the partial structure (I):

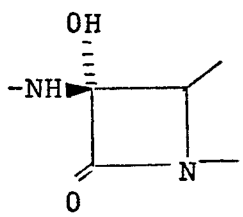

(I)

which process comprises reacting a β-lactam having the partial structure (II):

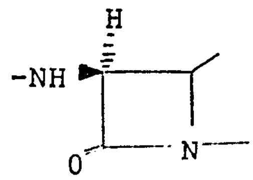

(II)

with an oxygenase enzyme and thereafter isolating the compound having partial structure (I) from the reaction mixture.

Preferably the compound having partial structure (II) is not a compound of formula (A) wherein $R^A$ is as hereinbefore defined.

Suitably the β-lactam having the partial structure (I) is a compound of formula (III) or a salt or ester thereof:

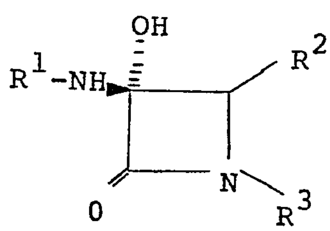

(III)

wherein $R^1$ is hydrogen or an acyl group, in particular that of an antibacterially active penicillin or cephalosporin;

$R^2$ is hydrogen or $C_{1-10}$ alkyl and $R^3$ is a sulphonic acid substituent $-SO_3H$;

or $R^2$ and $R^3$ together form a diradical of formula:

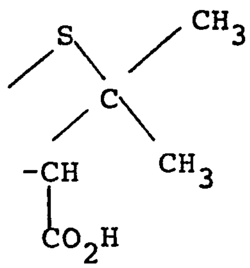
,
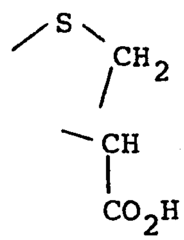

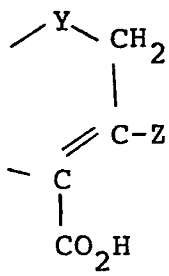

wherein Y represents oxygen or sulphur and Z represents hydrogen, halogen, $C_{1-4}$ alkoxy, $-CH_2Q$ or $-CH=CH-Q$ wherein Q represents hydrogen, halogen, hydroxy, mercapto, cyano, carboxy, carboxylic ester, carbanoyloxy $C_{1-4}$ alkyloxy, acyloxy, a heterocyclylthio group or a nitrogen containing heterocyclic group bonded via nitrogen.

Suitably $R^2$ and $R^3$ together represent $-S-C(CH_3)_2=CH(CO_2H)-$, $-S-CH_2-C(CH_2Q)=C(CO_2H)-$ or $-O-CH_2-C(CH_2Q)=C(CO_2H)-$ wherein Q is as hereinbefore defined.

Preferably $R^2$ and $R^3$ together represent $-S-C(CH_3)_2-CH(CO_2H)-$ and $-S-CH_2-C(CH_2Q)=CH(CO_2H)-$, ie when the compound of formula (III) are derivatives of a penicillin and cephalosporin.

A particularly preferred value for $R^2$ and $R^3$ together is $-S-C(CH_3)_2-CH(CO_2H)-$.

Suitable groups $R^1$ for inclusion in the compounds of the formula (III) include those of the sub-formulae (a) - (e):

$A_1 - (CH_2)_n - CH - (CH_2)_m - CO -$ (a)

X

$A_2 - CO -$ (b)

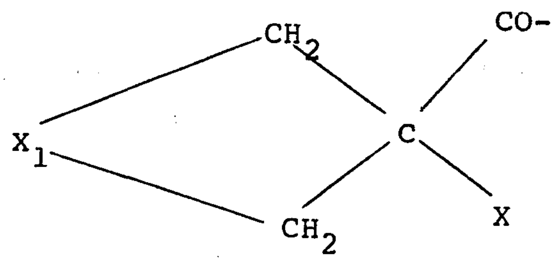

(c)

$A_2 - X_2 - (CH_2)_n - CO -$ (d)

$A_2 - C - CO -$

(e)

$N - OA_4$

wherein n is 0, 1 or 2; m is 0, 1 or 2; $A_1$ is $C_{1-6}$ alkyl, $C_{3-6}$ cycloalkyl, cyclohexenyl, cyclohexadienyl, an aromatic group, such as phenyl, substituted phenyl, thienyl or pyridyl or optionally substituted thiazolyl

group; X is a hydrogen or halogen atom, a carboxylic acid, carboxylic ester, sulphonic acid, azido, tetrazolyl, hydroxy, acylosy, amino, ureido, acylamino, heterocyclylamino, guanidino or acylureido group; $A_2$ is an aromatic group such as a phenyl, a 2,6-dimethoxyphenyl, 2-alkoxy-1-naphthyl, 3-arylisoxazolyl, 3-aryl-5-methylisoxazolyl group or a substituted alkyl group; $X_1$ is a $CH_2OCH2$, $CH_2SCH_2$ or $(CH_2)_n$ group; $X_2$ is an oxygen or sulphur atom; $A_3$ is an aryl or heteroaryl group such as phenyl, substituted phenyl or aminothiazolyl; and $A_4$ is hydrogen, $C_{1-6}$ alkyl, $C_{3-6}$ cycloalkyl, arylamino- carbonyl, $C_{1-6}$ alkylaminocarbonyl, $C_{1-6}$ alkanoyl, $C_{1-6}$ alkoxycarbonyl, $C_{2-6}$ alkenyl, carboxy $C_{1-6}$ alkyl, $C_{1-6}$ alkylsulphonyl and di-$C_{1-6}$ alkylphosphatomethyl.

More suitably $A_1$ is $C_{1-6}$ alkyl, $C_{3-6}$ cycloalkyl, cyclohexenyl, cyclohexadienyl, phenyl, hydroxy-phenyl, thienyl or pyridyl group; and X is a hydrogen or halogen atom, a carboxylic acid, carboxylic ester, azido, tetrazolyl, hydroxy, acyloxy, amino, ureido, guanidino or acylureido group.

Favoured groups $R^1$ for inclusion in the compounds of the formula (III) include those of the sub-formula (f) and (g):

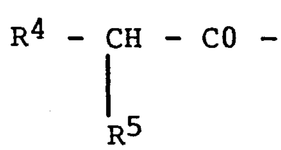

(f)

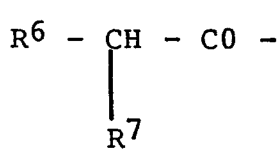

(g)

wherein $R^4$ is a phenyl, thienyl or phenoxy group; $R^5$ is a hydrogen atom or methyl group; $R^6$ is a phenyl, substituted phenyl, substituted thiazolyl, thienyl or cyclohexadienyl group; and $R^7$ is a hydroxyl, carboxylic acid group or lower alkyl or phenyl, tolyl or indanyl ester thereof, amino or a substituted amino group.

Suitably the substituted phenyl group for $R^6$ is a phenyl group sustituted with up to three groups selected from $C_{1-6}$alkyl, phenyl, halogen, $C_{1-6}$alkoxy, amino, nitro, hydroxy, $C_{1-6}$alkylamido, $C_{1-6}$alkylcarbonyloxy, carboxy, $C_{1-6}$alkoxycarbonyl, halo($C_{1-6}$)alkyl, oxo ($C_{1-6}$)alkyl, $C_{1-6}$alkylcarbonyl, aryloxy, arylcarbonyl, $C_{1-6}$alkylamino or di($C_{1-6}$)alkylamino.

Preferably $R^6$ is a phenyl, p-hydroxyphenyl, 3,4-dihydroxyphenyl, 3,4-diacetoxyphenyl, thienyl or cyclohexadienyl group.

Suitable values for Q in the compounds of the formula (III) include the acetoxy, heterocyclylthio group, and nitrogen containing heterocyclic group bonded via nitrogen.

More suitably Q represents the acetoxy or heterocyclylthio group.

The heterocyclylthio group may suitably be represented by the formula:

$$- S - Het$$

wherein αHetα is a five or six membered heterocyclic ring containing from 1 to 4 atoms selected from N, 0, and S unsubstituted or substituted with one or two

groups selected from $C_{1-6}$alkyl, $C_{1-6}$alkoxy, hydroxy; alkyl, $C_{1-6}$alkenyl, alkoxyalkyl, carboxyalkyl, sulphonylalkyl, carbamoylalkyl, trifluoromethyl, hydroxy, and halogen, oxo, (subst)aminoalkyl, carboxyalkyl.

Suitable salts of the compound of formula (III) will be pharmaceutically acceptable and include metal salts eg aluminium, alkali metal salts such as sodium or potassium alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)-amine or tris-(2-hydroxyethyl)-amine, cycloalkylamines such as dicyclohexylamine, or with procaine, dibenzylamine, N,N-dibenzylethylenediamine, 1-ephenamine, N-ethylpiperidine, N-benzyl-β-phenethylamine, dehydroabietylamine N,N'-bisdehydroabietylamine, ethylenediamine, or bases of the pyridine type such as pyridine, collidine or quinoline, or other amines which have been used to form salts with known penicillins and cephalosporins.

The choice of salt will be dependent on the remainder of the molecule with salts conferring water solubility being preferred.

Suitable esters of the compound of formula (III) are those which may be removed under conventional conditions. Such groups include benzyl, p-methoxybenzyl, benzoylmethyl, p-nitrobenzyl, 4-pyridylmethyl, 2,2,2-trichloroethyl, 2,2,2-tribromoethyl, t-butyl, t-amyl, allyl, diphenylmethyl, triphenylmethyl, adamantyl, 2-benzyloxyphenyl,

4-methylthiophenyl, tetrahydrofur-2-yl, tetrahydropyran-2-yl, pentachlorophenyl, p-toluenesulphonylethyl, methoxymethyl, or an *in vivo* hydrolysable ester radical.

One convenient intermediate within formula (III) is, the novel compound, 6β-(5-amino-5-carboxyvaleramido)-6α-hydroxy penicillanic acid (hereinafter referred to as 6α-hydroxy penicillin N):

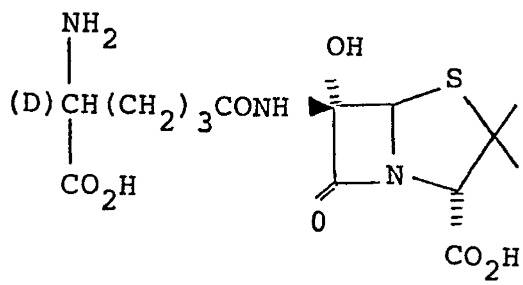

or a salt or ester thereof.

Suitably the compound having partial structure (I) will be in isolated form substantially free of proteinaceous material derived from the oxygenase or other protein present.

The salts of the compound having partial structure are a favoured aspect of this invention especially when substantially pure. The salts may be in crystalline or non-crystalline form; however, the crystalline forms are preferred.

When used herein the term "substantially pure" means at least 75% pure and preferably at least 85% pure, for example, 90-100% pure.

The oxygenase enzyme is suitably a dioxygenase of the type wherein one atom of the dioxygen molecule is

transferred to each of two substrate molecules. The two substrate molecules may be the same or different. The compound having partial structure (II) serves as one substrate molecule and suitably a molecule such as, for example, 2-oxo-glutaric acid serves as a second substrate (co-substrate) molecule. Those enzymes wherein 2-oxo-glutaric acid serves as co-substrate are preferred oxidases for use in the process of the present invention and are conveniently referred to as 2-oxo-glutaric acid coupled oxygenase.

From the foregoing it will be appreciated that the oxygenase enzymes utilise molecular oxygen and accordingly to facilitate reaction, preferably the reaction medium should be saturated with oxygen and high oxygen tension maintained over the reaction medium. Efficient transfer of oxygen from the gas phase into the solution phase is assisted by vigorous stirring or shaking of the reaction medium.

In addition to co-substrate and oxygen the oxidase enzyme reaction mixture will preferably contain a reducing agent such as dithiothreitol, ascorbate or 2-mercaptoethanol; and an additional source of iron ions, most suitably as ferrous sulphate.

A suitable source of the oxygenase enzyme is that produced by a microorganism. In particular it has been found that microorganisms of the Streptomyces genus, particularly those producing 7-methoxy-cephalosporins produce an oxygenase enzyme, which is suitable for the process of the present invention.

Preferred oxygenases are those produced by S. wadayamensis, S. lipmanii, S. griseus, S. clavuligerus, S. jumonjinensis and Nocardia lactamdurans. In particular, the following strains of these microorganisms are suitable: *S. wadayamensis* ATCC 21948, *S. lipmanii* NRRL 3584, *S. griseus* NRRL 3851, *S. clavuligerus* ATCC 27064, *S. jumonjinensis* NRRL 5741, *N. lactamdurans* NRRL 3802.

The oxygenase enzyme may be prepared by culturing the microorganism in a conventional manner, especially under aerobic conditions in a suitable liquid or semi-solid medium. In general, carbon and nitrogen sources which microorganisms can assimilate and inorganic salt nutrients essential for the growth of the microorganisms are included in the culture medium. In view of the nature of the prosthetic groups in oxidase enzymes the culture medium will contain a source of iron and where necessary molybdenum ions. The culture conditions may be a temperature of from 20°C to 80°C and pH of from 4 to 11. Preferred conditions are 20°C to 30°C at a pH of 5 to 9, suitably about pH7, for 1 to 10 days. The cultured microorganism containing the oxidase may be employed for the process of this invention in the form of the cultured broth, separated cells, or the oxidase may be isolated and used in purified form, partially purified form or as obtained in an impure state as a filtrate from a disrupted cell preparation.

Most suitably the enzyme is at least partially purified to remove other enzymes which might catalyse the destruction of the substrate molecules. The enzyme may be employed itself or attached to an insoluble support.

The hydroxylation reaction of the present invention is generally carried out in aqueous media, the reaction mixture being maintained in the range pH5 to 8, more

suitably 6.5 - 7.5 preferably about pH7. The pH is suitably controlled using buffers, such as, for example morpholino-propane sulphonic acid buffer at pH6.8. The temperature of the reaction should be suitable for the enzyme employed and is generally in the range 15°C to 40°C preferably ca. 30°C. The reaction time depends on such factors as concentrations of reactants, temperature and pH.

The enzyme substrate is suitably dissolved in buffer before mixing with the enzyme. The concentration of substrate solution will depend upon the solubility of the substrate; suitably the concentration of the substrate solution is in the range of 5% w/v. After reaction is complete the enzyme may be separated from the reaction mixture, suitably by acidification, which precipitates the enzyme, and the compound having partial structure (I) isolated by conventional methods. The initial purification of the derivative conveniently involves an ion exchange followed by a desalting chromatography.

In a further aspect of the present invention there is provided a process for the preparation of a β-lactam having partial structure (IV):

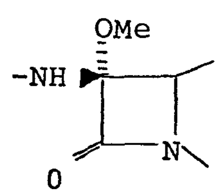

(IV)

which process comprises treating a compound having partial structure (I) with a methylating agent.

Suitable compounds having partial structure (I) include those of formula (III) hereinbefore.

The methylating agent may be chemical or enzymatic; preferably the methylating agent is a methyl transferase enzyme.

Suitable enzymatic methylating agents include those produced by a microorganism. In particular it has been found that microorganisms of the Streptomyces genus produces a methyl transferase which is suitable for the present process, particularly those species producing 7-methoxy cephalosporins.

The methyl transferase may suitably be prepared in a manner analogous to that used for the preparation of the oxygenase enzyme.

The enzymic methylation reaction of the present invention is generally carried out in aqueous media, the reaction mixture being maintained in the range pH5 to 8.5, more suitably 7 to 8 preferably from pH7.4 to 7.6. The pH is suitably controlled using buffers, such as, for example morpholinopropane sulphonic acid buffer at pH7.6. The temperature of the reaction should be suitable for the enzyme employed and is generally in the range 15°C to 40°C preferably ca. 30°C. The reaction time depends on such factors as concentrations of reactants, temperature and pH. After the reaction is complete the enzyme may be separated from the reaction mixture, suitably by acidification which precipitates the enzyme and the product isolated by conventional methods. The initial purification of the compound having partial structure (IV) conveniently involves an ion exchange followed by a desalting chromatography.

When the methylating agent is a methyl transferase enzyme the source of the methyl group is suitably S - adenosyl-methionine. In addition to the source of the methyl group the enzyme reaction mixture will preferably contain a reducing agent, such as dithiothreitol or 2-mercaptoethanol.

Preferably the compound having partial structure (I) is not a compound of formula (B) wherein $R^A$ is as hereinbefore defined and $R^B$ is hydroxy.

In a particularly preferred embodiment of the present invention the reaction of the compound having partial structure (II) with the oxygenase enzyme, and subsequently the reaction of the compound having partial structure (I) with the methyl transferase enzyme is carried out in a single reaction mixture without isolation of the compound having partial structure (I) with the proviso that the β-lactam having partial structure (I) is not a compound of formula (B) wherein $R^A$ is as defined hereinbefore and $R^B$ is hydroxy.

The following Examples serve to illustrate the present invention.

Example 1

Preparation of oxygenase

*Streptomyces wadayamensis* ATCC 21948 was spore inoculated into a seed flask (500 ml) containing 50 ml of the following medium:-

| | |
|---|---|
| glycerol | 10g |
| sucrose | 20g |
| tryptone (Oxoid) | 5g |
| yeast extract (Oxiod) | 1g |
| soyabean flour (Arkasoy 50) | 15g |
| $K_2HPO_4$ | 0.2g |
| deionised water | 1L |
| pH 6.8 | |

The flask was shaken at 240 rpm and at 26° for 48 hours. Production flasks (500 ml) containing 50 ml of production medium were inoculated with 5 ml from the seed flask. The production medium consisted of:-

| | |
|---|---|
| soluble starch | 30g |
| proline | 0.5g |
| sodium glutamate | 1.5g |
| NaCl | 5g |
| $MgSo_4$, $7H_20$ | 1g |
| $KH_2PO_4$ | 2g |
| $CaCl_2$ | 0.4g |
| $FeCl_3,6H_20$ | 0.1g |
| $MnCl_2,4H_2O$ | 0.1g |
| $ZnCl_2$ | 0.05g |
| deionised water | 1L |
| pH 6.8 | |

Production flasks were shaken (240 rpm) at 26° for 24 hours. The mycelium was harvested by centrifugation (12,000g for 10 mins at 4°C), washed with water and centrifuged again. The pellet of mycelium was re-suspended in 0.05M N-morpholinopropane sulphonic acid (MOPS) pH 7.5 at twelve times broth cell density. The suspension was cooled to 0°C and subjection to ultrasonic disintegration (M.S.E. 100W) for three periods of 15 seconds with 1 minute intervals. The mixture was centrifuged (27,000g for 30 minutes at 4°C) and the supernatant was subjectd to dialysis against water at 4°C for 5 hours. The dialysed solution constituted the enzyme preparation. The solution contained 10-15 mg protein/ml.

Example 2

Oxygenation of penicillin N

For the conversion of penicillin N to 6 -hydroxypenicillin N the reaction mixture contained penicillin N (2mM), $FeSO_4$ (1mM), ascorbic acid (1mM), 2-oxoglutaric acid (1mM), dithiothreitol (1mM), MOPS buffer pH 6.8 (50mM) and S. wadayamensis enzyme preparation (Example 1) (4-6mg protein per ml). The reaction mixture was shaken at 28°. After 2 hours the reaction was stopped by addition of glacial acetic acid (25µl per ml) and the precipitated protein was removed by centrifugation (3000g for 5 minutes). 6 -hydroxypenicillin N was recovered from the supernatant by ion exchange and reverse phase chromatography.

$\nu_{max}$ (KBr) 1760, 1650, 1600 $cm^{-1}$.
$\delta(D_2O)$ 1.46 (3H, s, $CH_3$, 1.48 (3H, s, $CH_3$), 1.6 - 2.0 (4H, complex, $CH_2CH_2$), 2.39 (2H, t, J 8Hz, $CH_2CO$), 3.72 (1H, t, J 7Hz, CHNH), 4.27(1H, s, C(3)H), 5.50 (1H, s, C(s)H)ppm.

Hplc: µ Bondapak C18 column eluted with 0.1M $NaH_2PO_4$ pH 3.2. Retention time 12.0 mins at a flow rate of 1ml/min.

In an analogous manner to that described above the following enzymatic conversions may be carried out:

(i) Penicillin G to 6α-hydroxypenicillin G;
(ii) 6β-[2-carboxy-2-thien-3'-ylacetamido penicillinic acid to 6α-hydroxy-6β-[2-carboxy-2-thien-3'-ylacetamido] penicillanic acid;

(iii) cephalothin to 7α-hydroxycephalothin;

(iv) cephalosporin C to 7α-hydroxycephalosporin C;

(v) desacetoxycephalosporin C to 7α-hydroxy desacetoxycephalosporin C; and

(vi) desacetylcephalosprin C to 7α-hydroxydesacetyl cephalosporin C.

Example 3

Preparation of methyl transferase

*Streptomyces clavuligerus* ATCC 27064 was spore inoculated into a seed flask (500 ml) containing 50 ml of the following medium:-

| | |
|---|---|
| glycerol | 10g |
| sucrose | 20g |
| Tryptone (Oxoid) | 5g |
| Yeast extract (Oxoid) | 1g |
| Soyabean flour (Arkasoy 50) | 15g |
| $K_2HPO_4$ | 0.2g |
| Deionised water | 1L |
| pH 6.8 | |

The flask was shaken at 240 rpm and at 26°C for 48 hours. Production flasks (500 ml) containing 50 ml of production medium were inoculated with 5 ml from the seed flask. The production medium consisted of:-

| | |
|---|---|
| soluble starch | 30g |
| proline | 0.5g |
| sodium glutamate | 1.5g |
| NaCl | 5g |
| $MgSO_4,7H_2O$ | 1g |
| $KH_2PO_4$ | 2g |
| $CaCl_2$ | 0.4g |
| $MnCl_2,4H_2O$ | 0.1g |
| $FeCl_3,6H_2O$ | 0.1g |
| $ZnCl_2$ | 0.05g |
| deionised water | 1L |
| pH 6.8 | |

Production flasks were shaken at 26° for 24 hours. The mycelium from two flasks was harvested by centrifugation (12,000g for 10 minutes at 4°C), washed with an equal volume of water and centrifuged again. The pellet was resuspended in MOPS buffer 0.05M pH 7.5 (4 ml). The cells were cooled to 0°C and subjected to ultrasonic disintegration (MSE, 100w) for three periods of 15 seconds with 1 minute intervals. The preparation was centrifuged (27,000g for 30 minutes at 4°C) and the supernatant was subjcted to dialysis against water for 5 hours. The dialysed supernatant constituted the enzyme preparation and contained 7-10 mg protein/ml.

0095320

Example 4

Methylation of 6α-hydroxypenicillin N

For the conversion of 6α-hydroxypenicillin N to 6α-methoxypenicillin N the reaction mixture contained 6α-hydroxypenicillin N (2mM), S-adenosylmethionine (1mM), dithiothreitol (1mM), MOPS buffer pH 7.6 (50mM) and S. clavuligerus ATCC 27064 enzyme preparation (Example 3) (3-4mg protein/ml). After incubation at 28° for 4 hours the reaction was stopped by the addition of glacial acetic acid (25μg/ml) and the precipitate was removed by centrifugation. 6α-methoxypenicillin N was removed from the supernatant by ion exchange and reverse phase chromatography.

δ($D_2O$) 1.50 (s,6H), 1.65 - 2.02 (m, 4H),
2.45 (t, 2H, J=6Hz, 3.52 (s, 3H),
3.55 - 3.85 (m, 1H), 4.30 (s, 1H), 5.59 (s, 1H).

Hplc: μ Bondapak C18 column eluted with 0.005M tetrabutylammonium phosphate pH 7.5. Retention time 6.8 minutes at a flow rate of 1.5ml/min.

In an analogous manner to that described above the following enzymatic conversions may be carried out:

(i) 6α-hydroxypenicillin G to 6α-methoxypenicillin G:
(ii) 6α-hydroxy-6β-[2-carboxy-2-thien-3'-ylacetamido penicillanic acid to 6α-methoxy-6β-[2-carboxy-2-thien-3'-ylacetamido]penicillanic acid;
(iii) 7α-hydroxycephalothin to 7α-methoxycephalothin;
(iv) 7α-hydroxycephalosporin C to 7α-methoxycephalosporin C;
(v) 7α-hydroxydesacetoxycephalosporin C to 7α-methoxydesacetoxycephalosporin C; and
(vi) 7α-hydroxydesacetylcephalosporin C to 7α-methoxydesacetylcephalosporin C.

Example 5

Oxygenation of cephaloporin C

Streptomyces clavuligerus ATCC 27064 was spore inculated into a seed flask (500ml) containing 50ml of the following medium:-

| | |
|---|---|
| glycerol | 10g |
| sucrose | 20g |
| Tryptone (Oxoid) | 5g |
| Yeast extract (Oxoid) | 1g |
| Soyabean flour (Arkasoy 50) | 15g |
| $K_2HPO_4$ | 0.2g |
| Deionised water | 1L |
| pH 6.8 | |

The flask was shaken at 240 rpm and at 26°C for 48 hours. Production flasks (500ml) containing 50ml of production medium were inoculated with 5ml from the seed flask. The production medium consisted of:-

| | |
|---|---|
| soluble starch | 30g |
| proline | 0.5g |
| sodium glutamate | 1.5g |
| NaCl | 5g |
| $MgSO_4$, $7H_2O$ | 1g |
| $KH_2PO_4$ | 2g |
| $CaCl_2$ | 0.4g |
| $MnCl_2,4H_2O$ | 0.1g |
| $FeCl_3,6H_2O$ | 0.1g |
| $ZnCl_2$ | 0.05g |
| deionised water | 1L |
| pH 6.8 | |

Production flasks were shaken at 26° for 24 hours.

The mycelium was harvested by centrifugation (12,000g for 10 mins) at 4°C, washed with water and centrifuged again. The cells were resuspended in 0.05M MOPS buffer, pH 7.5 containing dithiothreitol (1mM) at 12 times broth cell density. The suspension was cooled to 0°C and subjected to ultrasonic disintegration (MSE, 100W) for three periods of 15 seconds with one minute intervals. The sonicate was centrifuged (27,000g for 30mins) at 4°C and the supernatant was dialysed against water at 4°C for 5 hours. Dithiothreitol was added to the dialysed solution to a final concentration of 1mM. This solution which contained 7-10 mg protein/ml constituted the enzyme preparation.

For the conversion of cephalosporin C to 7α-hydroxycephalosporin C the reaction mixture contained cephalosporin C (1mM), ascorbic acid (1mM), 2-oxoglutaric acid (1mM), $FeSO_4$ (1mM), dithiothreitol (1mM), MOPS buffer pH 6.8 (50mM) and S.clavuligerus enzyme preparation (48ml) in a final volume of 120ml. The reaction mixture was shaken at 28°C. The reaction was stopped after 2 hours by the addition of glacial acetic acid (3ml) and the precipitated protein was removed by centrifigation. 7α-Hydroxycephalosporin C was recovered from the supernatant by ion exchange chromatography on Sephadex QAE followed by desalting on HP20 resin, as a white solid (2.6mg). The product was identical to synthetic 7α hydroxycephalosporin C.

$\nu_{max}$ (KBr) 1760, 1650(sh), 1600$cm^{-1}$

δ($D_2O$) 1.6-2.0(4H,m,C$\underline{H}_2$C$\underline{H}_2$), 2.07 (3H,s,C$\underline{H}_3$CO), 2.41(2H,t,J 6.7Hz, $CH_2$C$\underline{H}_2$CO), 3.29 and 3.65 (2H,ABq, J17.3Hz, C$\underline{H}_2$) 3.72 (1H,t,J 5.8Hz, $NH_2$C$\underline{H}$$CO_2$H), 4.65 and 4.81 (2H,ABq, J12.5, C$\underline{H}_2$), 5.09(1H,S,C$\underline{H}$).

$[M+Na]^+$ 476) (from +ve ion F.A.B. mass spectrum
)
$[M+H]^+$ 454)

Example 6

Methylation of 7α-hydroxycephalosporin C

For the conversion of 7α hydroxycephalosporin C to 7αmethoxycephalosporin C the reaction mixture contained 7α hydroxycephalosporin C (1mM), S-adenosylmethionine (1mM), dithiothreitol (1mM), MOPS buffer pH 7.6 (50mM) and S.clavuligerus ATCC 27064 enzyme preparation (Example 5) 3-4mg protein/ml. The reaction mixture was incubated at 28°C. The reaction was stopped after 3 hours by the addition of glacial acetic acid (25ul/ml of reaction mixture) and the precipitated protein was removed by centrifugation. 7α methoxycephalosporin C was recovered from the supernatant by ion exchange and reverse phase chromatography. The product was characterised by comparison with synthetic 7α methoxy-cephalosporin C.

Hplc : μ Bondapak C18 column eluted with 0.1M $NaH_2PO4$ pH 4.2. The retention time was 11.0 minutes at a flow rate of 1ml/minute.

νmax (KBr) 1760, 1720, 1610, 1410, 1240$cm^{-1}$. δ($D_2O$) 1.7-2.0(4H,m,$CH_2CH_2$), 2.03(3H,s,$CH_3CO$), 2.40(2H,t,J7Hz,$CH_2CO$), 3.20(1H,d,J18Hz,C(2)H), 3.46(3H,s,$OCH_3$), 3.60(1H,d,J18Hz,C(2)H), 3.67 (1H,t,J7Hz,$CHCH_2$), 4.60(1H,d,J12Hz,$CH_2OAc$), 4.82(1H,d,J12Hz,$CH_2OAc$), 5.11(1H,s,C(6)H)ppm.

Example 7

Oxygenation of cephalothin

*Streptomyces wadayamensis* ATCC 21948 was spore inoculated into a seed flask (500ml) containing 50ml of the following medium:-

| | |
|---|---|
| Glycerol | 10g |
| sucrose | 20g |
| tryptone (Oxoid) | 5g |
| yeast extract (Oxoid) | 1g |
| soyabean flour (Arkasoy 50) | 15g |
| $K_2HPO_4$ | 0.2g |
| deionised water | 1L |
| pH 6.8 | |

The flask was shaken at 240 rpm and at 26$^{O}$ for 48 hours. Production flasks (500ml) containing 50ml of production medium were inoculated with 5ml from the seed flask. The production medium consisted of:-

| | |
|---|---|
| soluble starch | 30g |
| proline | 0.5g |
| sodium glutamate | 1.5g |
| NaCl | 5g |
| $MgSO_4,7H_2O$ | 1g |
| $KH_2PO_4$ | 2g |
| $CaCl_2$ | 0.4g |
| $MnCl_2,4H_2O$ | 0.1g |
| $FeCl_3,6H_2O$ | 0.1g |
| $ZnCl_2$ | 0.05g |
| deionised water | 1L |
| pH 6.8 | |

Production flasks were shaken (240 rpm) at 26$^{O}$ for 24 hours.

The mycelium was harvested by centrifugation (12,000g for 10 mins) at 4°C, washed with water and centrifuged again. The cells were resuspended in 0.05M MOPS buffer pH 7.5 containing 1mM dithiothreitol at twelve times broth cell density. The suspension was cooled to 0°C and subjected to ultrasonic disintegration (MSE 100W) for three periods of 15 seconds with 1 minute intervals. The sonicate was centrifuged (27,000g for 30 mins) at 4°C and the supernatant was dialysed against water at 4°C for 5 hours. Dithiothreitol was added to the dialysed solution to a final concentration of 1mM. This solution which contained 10-15 mg protein/ml constituted the enzyme preparation.

For the conversion of cephalothin to 7α-hydroxycephalothin the reaction mixture contained cephalothin (1mM), $FeSO_4$ (1mM), ascorbic acid (1mM), 2-oxoglutaric acid (1mM) dithiothreitol (1mM) MOPS buffer pH 6.8 (50mM) and _S. wadayamensis_ enzyme preparation (4-6mg protein/ml). The reaction mixture was shaken at 28°C. After 2 hours the reaction was stopped by addition of glacial acetic acid (25 ul per ml reaction mixture) and the precipitated protein was removed by centrifugation (3,000g for 5 mins). 7α hydroxycephalothin was recovered from the supernatant by ion exchange chromatography and reverse phase chromatography. The product was identical to synthetic 7α hydroxycephalothin as shown by h.p.l.c. Using a C18 μ Bondapak column and eluting, with 0.05M potassium phosphate buffer pH 7.0/methanol (65:35) at 1ml/min the retention time was 5.6 mins. Cephalothin had a retention time of 7.4 mins. in the system. νmax(KBr) 1760,1740,1670,1600,1230$cm^{-1}$, δ($D_2O$) 2.09(3H,s,$CH_3CO$),3.23(1H,d,J18Hz, C(2)H), 3.60(1H,d,J18Hz, C(2)H), 3.91(2H,ABq collapsed, $CH_2CO$), 4.67(1H,d,J12Hz,$CH_2OAc$), 4.82(1H,d,J12Hz,$CH_2OAc$), 5.10 (1H,s,C(6)H),7.05(2H,m,arylH), 7.36(1H,m, aryl H)ppm. νmax ($H_2O$) 261(ε 7,400), 233(ε 12,500)nm.

Example 8

Methylation of 7α hydroxycephalothin

Streptomyces lipmanii NRRL 3584 was spore inoculated into a seed flask (500ml) containing 50ml of the following medium:-

| | |
|---|---|
| glycerol | 10g |
| sucrose | 20g |
| tryptone (Oxoid) | 5g |
| yeast extract (Oxoid) | 1g |
| soyabean flour (Arkasoy 50) | 15g |
| $K_2HPO_4$ | 0.2g |
| deionised water | 1L |
| pH 6.8 | |

The flask was shaken at 240 rpm and at 26° for 48 hours. Production flasks (500ml) containing 50ml of production medium were inoculated with 5ml from the seed flask. The production medium consisted of:-

| | |
|---|---|
| soluble starch | 30g |
| proline | 0.5g |
| sodium glutamate | 1.5g |
| NaCl | 5g |
| $MgSO_4,7H_2O$ | 1g |
| $KH_2PO_4$ | 2g |
| $CaCl_2$ | 0.4g |
| $MnCl_2,4H_2O$ | 0.1g |
| $FeCl_3,6H_2O$ | 0.1g |
| $ZnCl_2$ | 0.05g |
| deionised water | 1L |
| pH 6.8 | |

Production flasks were shaken (240 rpm) at 26° for 24 hours.

The mycelium was harvested by centrifugation (12,000g for 10 mins) at $4^0C$, washed with water and centrifuged again. The cells were resuspended in 0.05M MOPS buffer pH 7.5 containing 1mM dithiothreitol, at twelve times the broth cell density. The suspension was cooled to $0^0C$ and subjected to ultrasonic disintegration (MSE, 100W) for three periods of 15 seconds with 1 minute intervals. The sonicate was centrifuged (27,000g for 30 minutes) at $4^oC$ and the supernatant was dialysed against water at $4^0C$ for 5 hours. Dithiothreitol was added to the dialysed solution to a final concentration of 1mM. The solution which contained 10-15 mg protein/ml constituted the enzyme preparation.

For the conversion of 7α hydroxycephalothin to 7α methoxycephalothin the reaction mixture contained 7α hydroxycephalothin (1mM), S-adenosylmethionine (1mM), dithiothreitol (1mM) MOPS buffer pH 7.6 (50mM) and S.lipmanii enzyme preparation (4-6mg protein/ml). The reaction mixture was incubated at $28^0$. The reaction was stopped after 3 hours by the addition of glacial acetic acid (25ul per ml) and the precipitated protein was removed by centrifugation. 7α methoxycephalothin was recovered from the supernatant by ion exchange and reverse phase chromatography. The product was characterised by comparison with synthetic 7α methoxy cephalothin.

Hplc : μ Bondapak C18 column eluted with 0.05M potassium phosphate pH 7.0/acetonitrile (7:3).

The retention time of 7α methoxy cephalothin was 8.6 mins at 1ml/min.

$\nu$max(KBr)1760, 1740, 1680, 1610, 1230$cm^{-1}$. $\delta(D_2O)$ 2.09 (3H,s,C$\underline{H}_3$CO), 3.26(1H,d,$\underline{J}$18Hz, C(2)$\underline{H}$), 3.51(3H,s,OC$\underline{H}_3$), 3.63(1H,d,$\underline{J}$18Hz,C(2)$\underline{H}$), 3.97(2H,s,C$\underline{H}_2$CO), 4.68(1H,d,$\underline{J}$ 12Hz,C$\underline{H}_2$OAc), 4.83(1H,d,$\underline{J}$12Hz, C$\underline{H}_2$OAc), 5.15(1H,s,C(6)$\underline{H}$), 7.04(2H,m,aryl $\underline{H}$), 7.37 (1H,m,aryl $\underline{H}$) ppm. $\nu$max ($H_2O$) 160(ε 7,900), 234 (ε 12,700)nm.

Example 9

Oxygenation of 3-carbamoyloxymethyl-7β-(2'thienyl acetamido)ceph-3-em-4-carboxylate, sodium salt.

For the conversion of 3-carbamoyloxymethyl-7β-(2'-thienyl- acetamido)ceph-3-em-4-carboxylate, sodium salt to the 7α hydroxy derivative the reaction mixture contained 3-carbamoyloxymethyl-7β-(2'-thienyl-acetamido)ceph-3-em-4-carboxylate, sodium salt (lmM), ascorbic acid (lmM), 2-oxoglutaric acid (lmM), $FeSO_4$ (lmM), dithiothreitol (lmM), MOPS buffer pH 6.8 (50mM) and Streptomyces lipmanii enzyme preparation (Example 8) (4-6mg protein/ml). The reaction mixture was shaken at 28$^0$C. The reaction was stopped after 2 hours by the addition of glacial acetic acid (25ul per ml) and the precipitated protein was removed by centrifugation. The 7α hydroxyderivative of 3-carbamoyloxymethyl-7β-(2'-thienyl- acetamido)ceph-3-em-4-carboxylate, sodium salt was recovered from the supematant by ion exchange and reverse phase chromatography, and shown to be identical to the synthetic 7α hydroxyderivative of 3-carbamoyloxymethyl-7β-(2'-thienyl-acetamido)ceph-3-em-4-carboxylate, sodium salt.

Hplc: u Bondapak C18 column eluted with 0.05M potassium phosphate pH7/methanol (8:2). The retention time was 7.1 minutes at a flow rate of lml/min.

Example 10

Methylation of 6α hydroxybenzylpenicillin.

The conversion of 6α hydroxybenzylpenicillin to 6α methoxybenzylpenicillin was performed using the *S lipmanii* enzyme preparation and process similar to that described in Example 8. 6α methoxybenzylpenicillin was characterised by comparison with synthetic 6αmethoxybenzylpenicillin.

Hplc: μBondapak C18 column eluted with 0.05M potassium phosphate buffer/methanol (65:35). Retention time 8.4 mins at 1ml/min.

νmax (KBr) 1760, 1670, 1600, 1100$cm^{-1}$
δ($D_2O$) 1.36(3H,s,$CH_3$), 1.40(3H,s,$CH_3$), 3.45(3H,S,$OCH_3$), 3.65(2H,s,$CH_2CO$), 4.22(1H,s,C(3)H), 5.50(1H,s,C(5)H), 7.32(5H,s,aryl H)ppm.

Example 11

Methylation of sodium 6β-L-(4-amino-4-carboxy-butyramido)-6α hydroxypenicillanate.

The conversion of sodium 6β-L-(4-amino-4-carboxy-butyramido)-6α hydroxypenicillanate to the 6α methoxyderivative was performed utilising a process similar to that described in Example 8. The product was identified by comparison with synthetic 6α methoxyderivative.

Hplc: C18 μ Bondapak column eluted with 0.1M $NaH_2PO_4$pH 3.2
Retention time 7.0min at a flow rate of 1ml/min.

νmax (KBr) 1760, 1670, 1600$cm^{-1}$.
δ($D_2O$)1.47(3H,s,$C\underline{H}_3$), 1.49(3H,s,$C\underline{H}_3$), 2.15(2H,m,$CHC\underline{H}_2CH_2$), 2.54(2H,m,$C\underline{H}_2CO$), 3.51(3H,s,$OC\underline{H}_3$), 3.77(1H,t,$\underline{J}$7Hz,$C\underline{H}CH_2$), 4.29(1H,s,C(3)$\underline{H}$), 5.55(1H,s,C(5)$\underline{H}$)ppm.

Example 12

Methylation of sodium 6β-D-(4-amino-4-carboxybutyramido)-6α-hydroxypenicillanate.

The conversion of sodium 6β-D-(4-amino-4-carboxybutyramido)-6α-hydroxypenicillanate to the 6α methoxyderivative was performed utilising a process similar to that described in Example 8. The product was identified by comparison with synthetic 6α methoxyderivative.

Hplc : C18 μ Bondapak column eluted with 0.1M $NaH_2PO_4$ pH 3.2. Retention time 6.8min at a flow rate of 1ml/min.

νmax(KBr) 1760, 1670, 1600, 1100$cm^{-1}$. δ($D_2O$) 1.47(3H,s,$C\underline{H}_3$), 1.50(3H,s,$C\underline{H}_3$), 2.16(2H, m,$CHC\underline{H}_2CH_2$), 2.56(2H,t,$\underline{J}$7Hz,$C\underline{H}_2CO$), 3.51 (3H,s,$OC\underline{H}_3$), 3.80(1H,t,$\underline{J}$7Hz,$C\underline{H}CH_2$), 4.29 (1H,s,C(3)$\underline{H}$), 5.55(1H,s,C(5)$\underline{H}$)ppm.

Example 13

Methoxylation of potassium 6β-D-(5-amino -5-carboxyvaleramido)-2β-acetoxymethyl-2α-methyl-penam-3-carboxylate.

For the conversion of potassium 6β-D-(5-amino-5-carboxyvaleramido)-2β-acetoxymethyl-2α-methyl-penam-3-carboxylate to the 6αmethoxy derivative the reaction mixture contained potassium 6β-D-(5-amino-5-carboxyvaleramido)-2β-acetoxymethyl-2α-methyl-penam-3-carboxylate (2mM), ascorbic acid (1mM), 2-oxoglutaric acid (1mM), $FeSO_4$ (1mM), dithiothreitol (1mM), S-adenosylmethionine (1mM), MOPS buffer pH 7.5 (50mM) and S. wadayamensis enzyme preparation (Example 7) (4-6mg protein/ml). The reaction mixture was shaken at 28$^0$C. The reaction was stopped after 3 hours by the addition of glacial acetic acid (25ul per ml) and the precipitated protein was removed by centrifugation. The product was recovered from the supernatant by ion exchange and reverse phase chromatography.

Hplc : C18 uBondapak column eluted with 0.05M potassium phosphate buffer pH 7.0/methanol (98:2). Retention time 9.8 mins at a flow rate of 1ml/min.

Example 14

Oxygenation of deacetoxycephalosporin C

The conversion of deacetoxycephalosporn C to 7α hydroxydeacetoxycephalosporin C was carried out using the process described in Example 7. The product was characterised by comparison with synthetic 7α hydroxydeacetoxycephalosporin C.

Hplc : C18 μ Bondapak column eluted with 0.1M $NaH_2PO_4$ pH 4.2. Retention time 6.0 mins at a flow rate of 1ml/min.

δ($D_2O$) 1.6-2.0(4H,m,$C\underline{H}_2C\underline{H}_2$), 1.90(3H,s,$C\underline{H}_3$), 2.41(2H,M,$C\underline{H}_2CO$), 3.13(1H,d,$\underline{J}$18Hz,C(2)$\underline{H}$), 3.55(1H,d,$\underline{J}$18Hz, C(2)$\underline{H}$), 3.72(1H,t,$\underline{J}$7Hz, $C\underline{H}CH_2$), 5.06(1H,s,c(6)$\underline{H}$)ppm.

Example 15

Oxygenation of deacetylcephalosporin C

The conversion of deacetylcephalosporin C to 7α hydroxydeacetylcephalosporin C was carried out using the process described in Example 7. The product was characterised by comparison with 7α hydroxy-deacetylcephalosporin C prepared as in preparation 15a.

Hplc : C18 μBondapak column eluted with 0.1M $NaH_2PO_4$ pH 3.2. Retention time 4.1mins at a flow rate of 1ml/min.

Preparation 15a

Deacetylation of 7α-hydroxycephalosporin C with citrus esterase enzyme.

7α-hydroxycephalosporin C (5mg) was dissolved in MOPS buffer pH 7.0, 0.05M (20ml). Acetylesterase (Sigma EC 3.1.1.6), (0.2ml) was added and the mixture was stirred at 28°C for 3 hours. The solution was acidified with acetic acid (0.5ml) and loaded on a column (4ml) of QAE Sephadex A25 in the chloride form. The column was washed with water (16 ml) and eluted with 0.2M NaCl. Fractions (1ml) were collected and monitored for product by u.v. spectroscopy and h.p.l.c. ($C_{18}$ uBondapak, 0.1M $NaH_2PO_4$ pH 4.2)*. The fraction containing most deacetylated product (1ml) was submitted to preparative h.p.l.c. in the same solvent system, by means of multiple injections. The de-acetylated product was collected as it emerged from the h.p.l.c. detector. Pooled fractions were adjusted to pH 7.0 with sodium hydroxide.

* Retention times on h.p.l.c. (at a flowrate of 1ml/min):

| | |
|---|---|
| 7α-hydroxycephalosporin C | 16.4 minutes |
| desacetyl 7α-hydroxycephalosporin C | 3.6 minutes. |

Example 16

Oxygenation of 7β-aminocephalosporanic acid

For the conversion of 7β-aminocephalosporanic acid to 7α-hydroxy-7β-aminocephalosporanic acid the reaction mixture contained 7β-aminocephalosporanic acid (2mM), Fe $SO_4$ (1mM), ascorbic acid (1mM), 2-oxo-glutaric acid (1mM), dithiothreitol (1mM), MOPS buffer pH 6.8 (50mM) and S. wadayamensis enzyme preparation (4-6 mg protein/ml) Example 7). The reaction mixture was shaken at 28°C for 3 hours. The reaction was stopped by addition of glacial acetic acid (25 µl/ml) and the precipitated protein was removed by centrifugation. 7α-hydroxy-7β-aminocephalosporanic acid was recovered from the supernatant by ion exchange and reverse phase chromatography. The product was characterised by hplc (Retention time = 10.0 mins on a C18 µ Bondapak column eluted with 0.05M potassium phosphate buffer at a flow rate of 1ml/min) and by ultraviolet spectrophotometry.

Example 17

Methoxylation of 7β-aminocephalosporanic acid

For the conversion of 7β-aminocephalosporanic acid to 7α-methoxy-7β-aminocephalosporanic acid the reaction mixture contained 7β-aminocephalosporanic acid (2mM), $FeSO_4$ (1mM), ascorbic acid (1mM), 2-oxoglutaric acid (1mM), dithiothreitol (1mM), 5-adenosylmethionine (1mM), MOPS buffer pH 7.6 (50mM) and S.wadayamensis enzyme preparation (Example 7) (4-6mg/ml). The reaction mixture was shaken at 28°C for 3 hours. The reaction was stopped by addition of glacial acetic acid (25μl/ml) and the precipitated protein was removed by centrifugation. 7α-methoxy-7β-aminocephalosporanic acid was recovered from the supernatant by ion exchange and reverse phase chromatography. The product was characterised by hplc (Retention time = 12.0 mins on a C18 μ Bondapak column eluted with 0.05M potassium phosphate buffer pH 7.0 at a flow rate of 1ml/min).

Claims

1. A process for the preparation of a β-lactam having the partial structure (I):

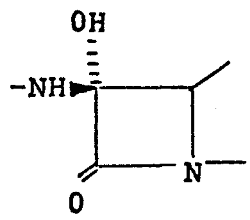

(I)

which process comprises reacting a β-lactam having the partial structure (II):

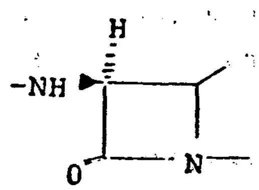

(II)

with an oxygenase enzyme and thereafter isolating the compound having partial structure (I) from the reaction mixture.

2. A process as claimed in claim 1 wherein β-lactam having the partial structure (I) is a compound of formula (III) or a salt or ester thereof:

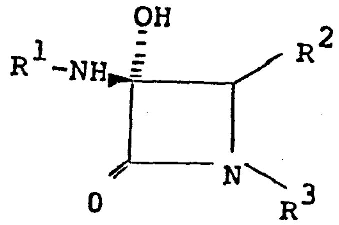

(III)

wherein $R^1$ is hydrogen or an acyl group, in particular that of an antibacterially active penicillin or cephalosporin;

$R^2$ is hydrogen or $C_{1-10}$ alkyl and $R^3$ is a sulphonic acid substituent $-SO_3H$;

or $R^2$ and $R^3$ together form a diradical of formula:

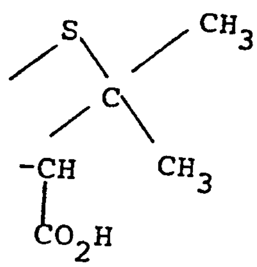

,

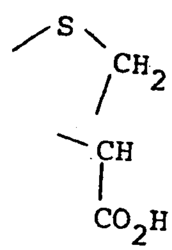

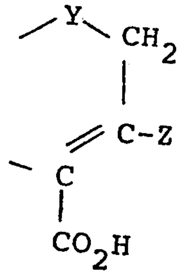

wherein Y represents oxygen or sulphur and Z represents hydrogen, halogen, $C_{1-4}$ alkoxy, $-CH_2Q$ or -CH=CH-Q wherein Q represents hydrogen, halogen, hydroxy, mercapto, cyano, carboxy, carboxylic ester, $C_{1-4}$ alkyloxy, acyloxy, a heterocyclylthio group or a nitrogen containing heterocyclic group bonded via nitrogen.

3. A process as claimed in claim 1 or claim 2 wherein the oxygenase enzyme is a dioxygenase of the type wherein one atom of the dioxygen molecule is transferred to each of two substrate molecules.

4. A process as claimed in any one of claims 1 to 3 wherein the dioxygenase is a 2-oxo-glutaric acid coupled oxygenase.

5. A process as claimed in any one of claim 1 to 4 wherein the oxygenase enzyme is that produced by a microorganisum of the Streptomyces genus.

6. A process for the preparation of a β-lactam having partial structure (IV):

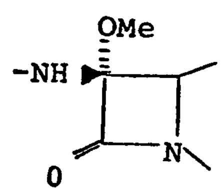

(IV)

which process comprises treating a compound having partial structure (I) with a methylating agent.

7. A process as claimed in claim 6 wherein the methylating agent is a methyl transferase enzyme produced by a microorganisum of the Streptomyces genus.

8. A process for the preparation of β-lactam having partial structure (IV) which comprises the reaction of the compound having partial structure (II) with an oxygenase enzyme, and subsequently the reaction of the compound having partial structure (I) with the methyl transferase enzyme in a single reaction mixture without isolation of the compound having partial structure (I) with the proviso that the β-lactam having partial structure (I) is not a compound of formula (B)

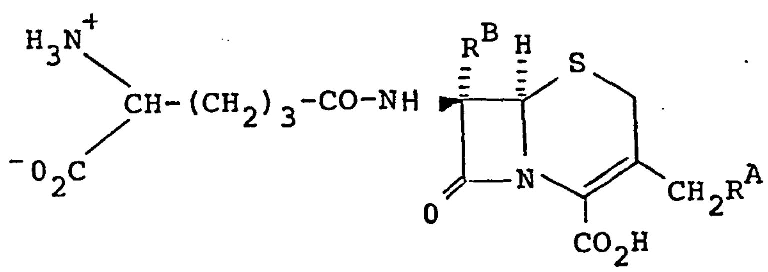

(B)

wherein $R^a$ represents $OCONH_2$, $OCOCH_3$ or H and $R^b$ is hydroxy.

9. 6β-(5-amino-5-carboxyvaleramido)-6α-hydroxy penicillanic acid.

10. 6β-(5-amino-5-carboxyvaleramido)-6α-methoxy penicillanic acid.

European Patent Office

# EUROPEAN SEARCH REPORT

0095320
Application number
EP 83 30 2844

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | GB-A-1 425 081 (FUJISAWA) * Claims * | 1,2 | C 12 P 37/00<br>C 12 P 35/08<br>C 12 P 17/10<br>C 07 D 499/00 //<br>C 12 P 35/00<br>C 12 R 1/465 |
| A | GB-A-1 382 316 (MERCK) * Claims * | 1,2 | |
| A | GB-A-1 463 468 (SANKYO) * Claims * | 1 | |
| A | US-A-4 123 612 (M. GORMAN) * Abstract; column 4, lines 49-68 * | 1 | |
| A | JOURNAL OF THE CHEMICAL SOCIETY, CHEMICAL COMMUNICATIONS, no. 17, 1981, pages 917-919 G. BAHADUR et al.: "Direct 1H N.M.R. observation of the cell-free conversion of delta-(L-alpha-aminoadipyl)-L-cysteinyl-D-valine and delta-(L-alpha-aminoadipyl)-L-cysteinyl-D-(-)-isoleucine into penicillins" * Whole article * | 1,9,10 | |

TECHNICAL FIELDS SEARCHED (Int. Cl. 3)

C 12 P 37/00
C 12 P 35/00
C 12 P 17/00
C 12 R 1/00
C 07 D 499/00

The present search report has been drawn up for all claims

| Place of search | Date of completion of the search | Examiner |
|---|---|---|
| THE HAGUE | 18-07-1983 | CHOULY J. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document